(19) Europäisches Patentamt / European Patent Office / Office européen des brevets

(11) EP 1 718 606 B1

(12) EUROPÄISCHE PATENTSCHRIFT

(45) Veröffentlichungstag und Bekanntmachung des Hinweises auf die Patenterteilung: **24.09.2014 Patentblatt 2014/39**

(21) Anmeldenummer: **05707300.9**

(22) Anmeldetag: **10.02.2005**

(51) Int Cl.:
***C07C 305/10*** $^{(2006.01)}$ ***C11D 1/29*** $^{(2006.01)}$
***A61Q 5/02*** $^{(2006.01)}$ ***A61Q 19/10*** $^{(2006.01)}$
***C09K 5/10*** $^{(2006.01)}$ ***A61K 8/46*** $^{(2006.01)}$

(86) Internationale Anmeldenummer: **PCT/EP2005/001319**

(87) Internationale Veröffentlichungsnummer: **WO 2005/077893 (25.08.2005 Gazette 2005/34)**

# (54) ALKYLETHERSULFATE

ALKYL ETHER SULFATES

SULFATES D'ÉTHERS D'ALKYLES

(84) Benannte Vertragsstaaten: **AT BE BG CH CY CZ DE DK EE ES FI FR GB GR HU IE IS IT LI LT LU MC NL PL PT RO SE SI SK TR**

(30) Priorität: **12.02.2004 DE 102004007152**

(43) Veröffentlichungstag der Anmeldung: **08.11.2006 Patentblatt 2006/45**

(73) Patentinhaber: **BASF SE**
**67056 Ludwigshafen (DE)**

(72) Erfinder:
- **TROPSCH, Jürgen**
  **67354 Römerberg (DE)**
- **ZELINSKI, Thomas**
  **67271 Neuleiningen (DE)**

(56) Entgegenhaltungen:
**EP-A- 0 167 337** **WO-A-94/11330**
**DE-A1- 4 436 066** **US-A- 3 843 706**

- **WEIL, JAMES K. ET AL.: "Oxypropylation of Fatty Alcohols, and the Sulfation Products" JOURNAL OF THE AMERICAN OIL CHEMISTS' SOCIETY, Bd. 43, 1966, Seiten 157-160, XP009049201**
- **SHINODA, KOZO ET AL: "Ionic surfactants soluble in hard water and in hydrocarbons: behavior of organized surfactant solutions as a function of the hydrophilic-lipophilic balance" JOURNAL OF PHYSICAL CHEMISTRY, Bd. 90, Nr. 7, 1986, Seiten 1228-30, XP002332576**
- **WEIL, JAMES K. ET AL: "Synthesis and surface active properties of long-chain ether alcohol sulfates R(OCH2CHR')iOSO3Na" CHIM. PHYS. APPL. PRAT. AG. SURFACE, C. R. CONGR. INT. DETERG., 5TH , MEETING DATE 1968, VOLUME 1, 45-50 PUBLISHER: EDICIONES UNIDAS, S. A., BARCELONA, SPAIN., 1969, XP001206849**
- **DATABASE CAPLUS [Online] CHEMICAL ABSTRACTS SERVICE, COLUMBUS, OHIO, US; MURATA, M. ET AL: "New anionic surfactants being resistant to hard water: sodium alkyl ether sulfates having oxypropylene groups" XP002332579 gefunden im STN Database accession no. 1981:499677 & COMUN. JORN. COM. ESP. DETERG., 12TH , 177-91 PUBLISHER: ASOC. INVEST. DETERGENTES, TENSIOACTIVOS AFINES, BARCELONA, SPAIN. CODEN: 46ARAB, 1981,**
- **MINANA-PEREZ, MATILDE ET AL: "Solubilization of polar oils with extended surfactants" COLLOIDS AND SURFACES, A: PHYSICOCHEMICAL AND ENGINEERING ASPECTS, Bd. 100, 1995, Seiten 217-24, XP002332578**

EP 1 718 606 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Alkylethersulfate aus alkoxylierten Alkoholen, die sulfoniert sind, wobei diese Alkylethersulfate zwischen der Alkoholkomponente und der Sulfatgruppe Propylen- und Ethylenoxid-Einheiten aufweisen, sowie die Verwendung dieser Alkylethersulfate in Wasch- und Reinigungsmitteln.

Aus dem Stand der Technik ist der Einsatz von Alkylethersulfaten, z.B. als Tenside bereits bekannt.

Die EP-A-1354872 betrifft ein Verfahren zur Herstellung von Salzen bestehend aus Aminen und Schwefelsäureestern. Die Schwefelsäureester können in der Seitenkette durchschnittlich 0.1 bis 10 Alkylenoxyeinheiten mit 2 bis 4 Kohlenstoffatomen tragen.

Die WO 00/58428 offenbart eine selbstverdickende, durch Hitze aktivierbare Reinigungszusammensetzung, welche anionische Tenside enthält, wie z.B. Alkylbenzolsulfate, ethoxylierte Alkylethersulfate und propoxylierte Alkylethersulfate.

Die WO 99/65972 betrifft eine wässrige Emulsion beinhaltend wenigstens ein Harz, das silikonhaltige Bausteine beinhaltet und ein Reaktionsprodukt aus Ammoniak oder polyfunktionellen aromatischen oder aliphatischen Aminen, Carbonsäuren oder -anhydriden und nicht-ionischen, anionischen oder amphoteren Tensiden ist. Die anionischen Tenside können dabei ethoxylierte und propoxylierte Derivate von Alkylsulfaten sein mit durchschnittlich 0.5 bis 10 Ethylen- und/oder Propylenoxid-Einheiten.

Die WO 95/15408 offenbart ein Verfahren zum Ätzen von Aluminium oder AluminiumLegierungen in einem kaustischen Bad, beinhaltend ein anionisches Tensid des Sulfat- oder Sulfonat-Typs.

Die JP 06017089 und JP 06017088 betreffen eine milchige Reinigungszusammensetzung mit perlmuttartigem Aussehen mit guter Langzeitstabilität und hervorragender Reinigungswirkung beinhaltend ein Alkylglykosid, ein anionisches Tensid und das Salz eines Schwefelsäureesters eines Alkohol-Propylenoxid-Addukts mit einem durchschnittlichen Molekulargewicht von 400 bis 4000.

US 3,843,706 A offenbart langkettige Ether auf Basis von Alkoholen und entsprechende Sulfate auf Basis von Alkoholen, welche Propylenoxid und 1,2-Butylenoxid enthalten, der allgemeinen Formel $R(OCH_2CH(R'))_nOY$, wobei R ein linearer Alkylrest mit 12 bis 18 Kohlenstoffatomen ist, R' ist ausgewählt aus Methyl und Ethyl, n ist eine ganze Zahl von 1 bis 4 und Y ist ausgewählt aus Wasserstoff und $SO_3M$, wobei M ausgewählt ist aus Natrium, Kalium, Lithium, Ammonium und $NH(C_2H_4OH)_3$, und deren Verwendung als bioabbaubare oberflächenaktive Substanzen.

In Weil, James K. et al., "Oxypropylation of fatty alcohols and the sulfation products", Journal of the American Oil Chemists' Society, Bd. 43, 1966, Seiten 157 bis 160 werden Verbindungen offenbart, die durch Umsetzung von $C_{12}$-, $C_{14}$-, $C_{16}$- und $C_{18}$-Alkoholen mit Propylenoxid erhalten werden. Pro Molekül der genannten Verbindungen liegen ein oder zwei Moleküle Propylenoxid vor. In einem weiteren Verfahrensschritt können die so erzeugten propoxylierten Alkohole sulfatiert werden.

Die Schrift Chinoda, Kozo et al., "Ionic surfactants soluble in hard water and in hydrocarbons: behaviour of organized surfactant solutions as a function of the hydrophiliclipophilic balance", Journal of Physical Chemistry, Bd. 90, Nr. 7, 1986, Seiten 1228 bis 1230 offenbart Alkyloxipropylensulfate, die gebildet werden durch Umsetzung von $C_{12}$-$C_{16}$-Alkoholen mit Propylenoxid und anschließender Sulfatierung.

In Weil, James K. et al., "Synthesis and surface active properties of long-chain ether alcohol sulfates", Chim. Phys. Appl. Prat. Ag. Surface, C. R. Congr. Int. Deterg., 5th, Meeting Date 1968, Vol. 1, 45 - 50, werden Verbindungen der Formel $R(OCH_2CHR')_iOSO_3Na$ genannt. Diese werden hergestellt durch Sulfatierung von isolierten Reaktionsprodukten aus Ethylen-, Propylen- und 1,2-Butylenoxid mit normalen primären Alkoholen mit 12, 14, 16 und 18 Kohlenstoffatomen.

In Database Caplus Online, Chemical abstracts service, Columbus, Ohio, US: Murata, M. et al., "New anionic surfactants being resistant to hard water: sodium alkyl ether sulfates having oxypropylene groups" werden oberflächenaktive Verbindungen der allgemeinen Formel $R(CH_2CHCH_3)_nOSO_3$ Na mit R = $C_{12}$-, $C_{16}$- oder $C_{18}$-Alkohol und n = 1,01 bis 5,24 offenbart.

Aufgabe der vorliegenden Erfindung ist das Bereitstellen von Alkylethersulfaten, die vorteilhaft als anionische Tenside in Wasch- und Reinigungsmitteln eingesetzt werden können.

Diese Aufgabe wird erfindungsgemäß gelöst von Alkylethersulfaten der allgemeinen Formel I

$$RO\text{-}(CH_2\text{-}CHR^1O)_y\text{-}(CH_2CH_2O)_zSO_3^- \, M^+ \qquad (I),$$

mit der Bedeutung

R von 2-Propylheptanol, i-$C_{13}$-Alkoholen oder Mischungen von 2-Propylheptanol und i-$C_{13}$-Alkoholen abgeleiteter Rest,

$R^1$ Methyl,

$M^+$ Kation, ausgewählt aus der Gruppe bestehend aus Alkalimetallen, $NH_4^+$ und $HNR^2{}_3^+$, wobei $R^2$ ausgewählt ist

aus der Gruppe bestehend aus linearen oder verzweigten Alkylresten, $CH_2CH_2OH$ und $CH_2CH(OH)CH_3$,

y mittlerer Wert 2,

z mittlerer Wert von 1 oder 3,

für die der Quotient A der kritischen Micellenkonzentration cmc

$$A = \frac{\text{cmc}\ (RO\text{-}(CH_2CH_2O)_zSO_3^-\ M^+)}{\text{cmc}\ (RO\text{-}CH_2\text{-}CHR^1O)_y\text{-}(CH_2CH_2O)_zSO_3^-\ M^+)} > 1$$

ist.

Die Erfindung betrifft eine bestimmte Auswahl aus den bekannten Alkylethersulfaten, die der allgemeinen Formel I entsprechen und die für A einen Wert ergeben, der größer als 1 ist.

Der Quotient

$$A = \frac{\text{cmc}\ (RO\text{-}(CH_2CH_2O)_zSO_3^-\ M^+)}{\text{cmc}\ (RO\text{-}(CH_2\text{-}CHR^1O)_y\text{-}(CH_2CH_2O)_zSO_3^-\ M^+)}$$

beschreibt das Verhältnis der cmc, d.h. die kritische Micellenkonzentration, von Alkylethersulfaten, die an den langkettigen Alkohol (RO-) anschließend ausschließlich Ethylenoxid-Einheiten oder keine Alkoxideinheiten gebunden aufweisen zu der cmc von Alkylethersulfaten, die zwischen dem langkettigem Alkohol (RO-) und der Sulfatgruppe gegebenenfalls Ethylenoxid und weitere, von Ethylenoxid verschiedene, Alkylenoxid-Einheiten ($CH_2$-$CHR^1O$) aufweisen.

Es wurde gefunden, dass Alkylethersulfate der allgemeinen Formel I, die einen Quotient A bilden, der größer 1 ist, bei ihrer Verwendung als Anionentensidkomponente in Wasch- und Reinigungsmitteln, in chemisch-technischen Anwendungen oder in kosmetischen Formulierungen besonders günstige Eigenschaften zeigen.

Daraus folgt, dass die erfindungsgemäßen Alkylethersulfate der allgemeinen Formel I, verglichen mit den entsprechenden Alkylethersulfaten, die gegebenenfalls ausschließlich Ethylenoxid-Einheiten oder keine Alkoxid-Einheiten aufweisen, einen kleineren Wert für die kritische Micellenkonzentration (cmc) aufweisen, woraus ein Wert von A resultiert, der größer 1 ist. Dieser Teil der Alkylethersulfate kann durch Synthese der erfindungsgemäßen Alkylethersulfate und Synthese der entsprechenden gegebenenfalls ausschließlich Ethylenoxid-Einheiten oder keine Alkoxid-Einheiten beinhaltenden Alkylethersulfate und Vergleich der kritischen Micellenkonzentrationen (cmc) ermittelt werden.

Die Werte für die kritische Micellenkonzentration (cmc) in mmol/l werden über Konzentrationsreihen mit der DeNuoy-Methode der Oberflächenspannungsmessung bestimmt.

Der das Verhältnis zwischen den Micellenkonzentrationen der Alkylethersulfate beinhaltend gegebenenfalls ausschließlich Etylenoxid-Einheiten und der erfindungsgemäßen Alkylethersulfate beschreibende Quotient A hat erfindungsgemäß einen Wert von größer 1 bis 100, bevorzugt hat A einen Wert von größer 1 bis 50, ganz besonders bevorzugt hat A einen Wert von größer 1 bis 20.

Der Quotient A ist in den zuvor genannten Grenzen bevorzugt größer 1.1, besonders bevorzugt größer 1.2 und ganz besonders bevorzugt größer 1.5.

Die erfindungsgemäßen Alkylethersulfate zeichnen sich dadurch aus, dass sie eine kritische Micellenkonzentration aufweisen, die mit der cmc von längerkettigen Alkoholen zu vergleichen ist.

Unter Micellen versteht man durch Assoziation gebildete Aggregate von gelösten Molekülen. Im engeren Sinn bezeichnet man als Micellen diejenigen Aggregate, die sich aus Tensid-Molekülen in wässrigen Lösungen oberhalb einer bestimmten Temperatur und einer charakteristischen Konzentration bilden. Diese Konzentration nennt man die kritische Micellenkonzentration (critical micell concentration, cmc). Das Erreichen der kritischen Micellenkonzentration gibt sich durch eine sprunghafte Änderung der physikalischen Eigenschaften zu erkennen. Beim Überschreiten der kritischen Micellenkonzentration bleibt die Molekülkonzentration in der Lösung praktisch konstant und die überschüssigen Moleküle bilden Micellen.

Daraus ergibt sich, dass die erfindungsgemäßen Alkylethersulfate durch ihre niedrigere kritische Micellenkonzentration bei einer geringeren Konzentration in wässriger Lösung Micellen bilden, die für eine gute Tensidwirkung notwendig sind. Dadurch kann durch die erfindungsgemäßen Alkylethersulfate die Einsatzmenge an Tensiden in Wasch- und Reinigungsmitteln gesenkt werden.

Betrachtet man die erfindungsgemäßen Beispiele 1 bis 6 und die entsprechenden Referenzbeispiele 1 bis 6,

so wird deutlich, dass die cmc-Werte der erfindungsgemäßen Alkylethersulfate 1 bis 6 durchgängig niedriger ausfallen als bei den entsprechenden Referenzbeispielen 1 bis 6, die jeweils nur Ethylenoxid und kein Propylenoxid bzw. Butylenoxid direkt an den entsprechenden Alkohol gebunden aufweisen.

Die erfindungsgemäßen Alkylethersulfate der allgemeinen Formel I können nach einer der folgenden Methoden hergestellt werden:

Die entsprechenden Alkoholalkoxidkomponenten können in die erfindungsgemäßen Alkylethersulfate der allgemeinen Formel I überführt werden, indem man sie in an sich bekannter Weise mit Schwefelsäure oder Schwefelsäurederivaten zu sauren Alkylethersulfaten sulfatiert.

Sulfatierungsreaktionen von Alkoholen sind bereits beschrieben worden z.B. in US-A-3,462,525, 3,420,875 oder 3,524,864. Details zur Durchführung dieser Reaktion finden sich auch in "Ullmann's Encyclopedia of Industrial Chemistry", 5.Auflage Bd. A25 (1994), Seiten 779 - 783 und in den dort angegebenen Literaturverweisen.

Wird Schwefelsäure selbst zur Veresterung eingesetzt, so verwendet man zweckmäßigerweise eine 75 bis 100 Gew.-%ige, vorzugsweise 85 bis 98 Gew.-%ige Säure (sog. "konzentrierte Schwefelsäure" oder "Monohydrat"). Die Veresterung kann in einem Lösungs- oder Verdünnungsmittel vorgenommen werden, wenn es für die Steuerung der Reaktion, z.B. die Wärmeentwicklung erwünscht ist. In der Regel wird der alkoholische Reaktant vorgelegt, und das Sulfatierungsmittel unter ständigem Durchmischen allmählich zugefügt. Wenn eine vollständige Veresterung der Alkoholalkoxidkomponente gewünscht wird, verwendet man das Sulfatierungsmittel und die Alkoholalkoxidkomponente im Molverhältnis von 1:1 bis 1:1.5, vorzugsweise von 1:1 bis 1:1.2. Geringere Mengen von Sulfatierungsmittel können vorteilhaft sein, wenn Mischungen von Alkoholalkoxylaten eingesetzt werden. Die Veresterung wird normalerweise bei Temperaturen von 25 bis 85°C, vorzugsweise im Bereich von 45 bis 75°C, durchgeführt.

Gegebenenfalls kann es zweckmäßig sein, die Veresterung in einem niedrig siedenden, mit Wasser nicht mischbaren Lösungs- und Verdünnungsmittel bei dessen Siedepunkt auszuführen, wobei das bei der Veresterung entstehende Wasser azeotrop abdestilliert wird.

Anstelle von Schwefelsäure der oben angegebenen Konzentration kann zur Sulfatierung der erfindungsgemäßen Alkanolgemische auch beispielsweise Schwefeltrioxid, Schwefeltrioxid-Komplexe, Lösungen von Schwefeltrioxid in Schwefelsäure ("Oleum"), Chlorsulfonsäure, Sulfurylchlorid oder auch Amidosulfosäure eingesetzt werden. Die Reaktionsbedingungen sind dann entsprechend anzupassen.

Wird Schwefeltrioxid als Sulfatierungsmittel eingesetzt so kann die Reaktion auch vorteilhafterweise in einem Fallfilmreaktor im Gegenstrom oder Gleichstrom, gegebenenfalls auch kontinuierlich, ausgeführt werden.

Die Ansätze werden nach der Veresterung durch Alkalizusatz neutralisiert und, gegebenenfalls nach Entfernung überschüssigen Alkalisulfates und eventuell vorhandener Lösungsmittel, aufgearbeitet.

Wird Chlorsulfonsäure als sulfatierendes Reagenz eingesetzt, so wird die entsprechende Alkoholalkoxidkomponente in einer Rührapparatur unter inerten Bedingungen vorgelegt. Unter starkem Rühren wird eine entsprechende Menge Chlorsulfonsäure zugetropft. Das Molverhältnis zwischen Alkoholkomponente und Chlorsulfonsäure liegt bei 0.5:1 bis 1:0.5, bevorzugt liegt das Verhältnis bei 0.75:1 bis 1:0.75. Ganz besonders bevorzugt liegt das Molverhältnis von Alkoholalkoxidkomponente zu Chlorsulfonsäure bei 1:1. Nach Entfernen des HCl-Gases wird der Reaktionsansatz mit Natronlauge auf einen leicht alkalischen pH-Wert eingestellt.

Die Erfindung betrifft weiterhin auch die Verwendung der erfindungsgemäßen Alkylethersulfate als Anionentensidkomponente in Wasch- und Reinigungsmitteln.

Die erfindungsgemäßen Alkylethersulfate können in Wasch- und Reinigungsmitteln als alleinige Anionentensidkomponente, aber auch in Kombination mit anderen Anionentensiden, mit den üblichen Bestandteilen eingesetzt werden.

Bevorzugte Ausführungsformen der Wasch- und Reinigungsmittel sind Pulverwaschmittel, Kompaktwaschmittel, Superkompaktwaschmittel, Waschmittelextrudate, Waschmittelgele, Flüssigwaschmittel, Flüssigwaschmittelkapseln ("pouches"), Flüssigwaschmittelkonzentrate, Handgeschirrspülmittel, Geschirrspülmittel für maschinelle Geschirrspüler, Scheuerreiniger oder -milch, Handwaschpasten oder -gele, Allzweckreiniger, Glasreiniger, Fensterreiniger, Bodenreiniger, Badreiniger, WC-Reiniger, Küchenreiniger, Schlachthausreiniger, Autoshampoos oder Metallreiniger.

Insbesondere können die erfindungsgemäßen Alkylethersulfate als Anionentensidkomponente in Waschmitteln oder Handgeschirrspülmitteln eingesetzt werden.

Wasch- und Reinigungsmittel dieser Art sind im Stand der Technik vielfach beschrieben worden. Einen sehr guten Überblick über die Wirkungsweise und die Zusammensetzung von Reinigungs- und Waschmitteln findet man beispielsweise in Ullmann's Encyclopedia of Industrial Chemistry, 5. Auflage, Bd. A7, (1986), Seiten 137 ff. Die Wasch- und Reinigungsmittel enthalten ein Tensid oder mehrere Tenside aus gleichen oder unterschiedlichen Tensidgruppen und in der Regel weitere Hilfs- und Zusatzstoffe, die entweder zur Konfektionierung erforderlich sind und/oder die einer Anpassung der Reinigungs- und Waschmittel an den geplanten speziellen Verwendungszweck oder die Art der Anwendung (Reinigung von Hand oder Maschinen) dienen. Bestandteile, die neben den verschiedenen Tensiden in wechselnden Kombinationen und Anteilen in vielen Reinigungs- und Waschmitteln eingesetzt werden können, sind zum Beispiel Builder (Sequestrierungsmittel) und Co-Builder, pH-Regulatoren, wie anorganische oder organische Säuren, anorganische oder organische Basen und Puffersysteme, Dispergiermittel, Verdickungsmittel, Enzyme, Bleichsysteme, hydrotrope Verbindungen als Lösungsvermittler bzw. Solubilisatoren, wie z.B. Harnstoff oder Alkohole, organische Lösemittel, feinteilige Abrasivkomponenten, wie z.B. Quarz- oder Marmormehl, Kreide, Diatomeenerde, Bimsstein, Polierrot oder Schmirgel, Schaumregulatoren zur Stabilisierung oder Dämpfung des Schaums, Haut- und Korrosionsschutzmittel, desinfizierende Verbindungen oder Systeme, beispielsweise solche, die Jod enthalten oder die Chlor oder unterchlorige Säure freisetzen, wie z.B. Dichlorisocyanurat, Parfüm, Farbstoffe, und Biozide. Wesentlicher Anteil an der Reinigungswirkung der im Stand der Technik beschriebenen Wasch- und Reinigungsmittel kommt den darin enthaltenen Tensiden zu. Verwendung finden ionische Tenside und zwar sowohl anionische wie beispielsweise Alkoholsulfate, Alkylethersulfate, Alkylbenzolsulfonate, $\alpha$-Olefinsulfonate, Sulfosuccinate als auch kationische Tenside, wie beispielsweise $C_8$ bis $C_{16}$-Dialkyldimethylammoniumhalogenide, Dialkoxydimethylammoniumhalogenide oder Imidazoliniumsalze mit langkettigem Alkylrest.

Auch der Einsatz von amphoteren Tensiden, beispielsweise von Derivaten von sekundären oder tertiären Aminen wie z.B. $C_6$-$C_{18}$-Alkylbetainen oder $C_6$-$C_{15}$-Alkylsulfobetainen oder Aminoxiden wie Alkyldimethylaminoxiden ist bereits beschrieben worden.

Auch nichtionische Tenside, insbesondere auch Alkoxylate und Polyglycoside von längerkettigen und langkettigen Alkanolen insbesondere mit 8 bis 20 C-Atomen sowie Alkoxylate von Alkylaminen und Alkylamiden werden in Wasch- und Reinigungsmitteln eingesetzt. Es ist insbesondere auch bekannt, Oxoalkohole mit 10 bis 13 C-Atomen in Form ihrer Phosporsäure- oder Schwefelsäureester sowie Alkoxylate dieser Oxoalkohole direkt oder in Form ihrer Phosporsäure- oder Schwefelsäureester als Tenside in Wasch- und Reinigungsmitteln einzusetzen.

Im Interesse eines möglichst sparsamen Stoffeinsatzes, hoher Wirtschaftlichkeit und geringer Umweltbelastung streben die Reinigungs- und Waschmittelhersteller nach einer stetigen Verbesserung der Wirksamkeit ihrer Produkte und insbesondere der darin enthaltenen Tenside.

Es wurde nun gefunden, dass die oben beschriebenen erfindungsgemäßen Alkylethersulfate als Aniontensidkomponente in Reinigungs- und Waschmitteln gegenüber bekannten Mitteln eine erheblich überlegene Wirksamkeit entfalten.

Ein weiterer Gegenstand der vorliegenden Erfindung ist somit die Verwendung der vorstehend beschriebenen Alkylethersulfate der allgemeinen Formel I als Anionentensidkomponente in Wasch- und Reinigungsmitteln. Des Weiteren beinhalten die Wasch- und Reinigungsmittel übliche bekannte Hilfs- und Zusatzstoffe und gegebenenfalls zusätzliche Tenside.

Der Mindestanteil der Alkylethersulfate der allgemeinen Formel I am Gesamtgewicht der erfindungsgemäßen Wasch- und Reinigungsmittel wird so bemessen, dass sich eine signifikante Wirkung dieses Zusatzes zeigt.

Zweckmäßigerweise wird der Anteil der erfindungsgemäß einzusetzenden Alkylethersulfate so eingestellt, dass sich im Zusammenwirken mit den übrigen Bestandteilen des Wasch- und Reinigungsmittels eine optimale Reinigungswirkung ergibt. In der Regel wird eine gute Reinigungswirkung erreicht, wenn der Anteil der erfindungsgemäßen Alkylethersulfate der Formel I in dem Wasch- und Reinigungsmittel, bezogen auf das Gesamtgewicht des Mittels, 0.01 bis 40 Gew.-%, vorzugsweise 0.1 bis 35 Gew.-%, insbesondere 0.1 bis 30 Gew.-% beträgt.

Die Verbindungen der allgemeinen Formel I können einheitliche Substanzen sein, sie können aber auch Gemische darstellen, in denen verschiedene unter die allgemeine Formel I fallende Substanzen miteinander gemischt sind. Die Komponenten dieser Gemische können sich bezüglich der Bedeutungen von R, $R^1$ und M, und hinsichtlich der Werte von y und z unterscheiden. Dies hat zur Folge, dass die bei der Elementaranalyse der erfindungsgemäßen Alkylethersulfate der allgemeinen Formel I erhaltenen analytischen Werte, beispielsweise die für die Oxoalkohol-Baugruppe erhaltenen C-und H-Werte, und insbesondere die Werte der Alkoxygruppen-Bestimmung bei der Rückrechnung auf die Strukturformel zu gebrochenen Werten für y und z führen. Die mittleren Werte für y und z sind die Mittelwerte der Alkoxylierungsgrade der in den Mischungen vorhandenen Verbindungen. Diese Werte sind bei Proben enthaltend nur eine Verbindung ganzzahlige Werte. Selbstverständlich stellen auch derartige Substanzgemische unter die allgemeine Formel I fallende erfindungsgemäße Verbindungen dar, die die beschriebenen Vorteile gegenüber dem Stand der Technik aufweisen.

Selbstverständlich werden die Zusammensetzungen der Reiniger den verschiedenen Zwecken angepasst, wie es dem Fachmann aus dem Stand der Technik geläufig ist. Hierzu können den die erfindungsgemäßen Alkylethersulfate der allgemeinen Formel I enthaltenden Wasch- und Reinigungsmitteln alle zweckentsprechenden aus dem oben genannten Stand der Technik bekannten Hilfs- und Zusatzstoffe zugefügt werden.

In vielen Fällen ist es zweckmäßig, die erfindungsgemäß eingesetzten Alkylethersulfate der allgemeinen Formel I mit anderen nichtionischen Tensiden, wie z.B. Alkoholalkoxilaten, Alkylaminalkoxilaten, Alkylamidalkoxilaten, Alkylpolyglucosiden, oder mit ionischen, vorzugsweise anionischen, Tensiden, wie z.B. längerkettigen oder langkettigen von den erfindungsgemäßen Alkylethersulfaten verschiedenen Alkoholsulfaten, Alkylbenzolsulfonaten, $\alpha$-Olefinsulfonaten, Sulfosuccinaten, oder mit amphoteren Tensiden, wie z.B. Alkylaminoxiden, oder Betainen zu kombinieren.

Im Folgenden werden Beispiele für zur Kombination geeigneter Tenside unterschiedlicher Natur genannt:

Als nichtionische Tenside eignen sich beispielsweise alkoxylierte lineare oder verzweigte $C_8$- bis $C_{22}$-Alkohole wie Fettalkoholalkoxylate oder Oxoalkoholalkoxylate. Die Alkoxylierung kann mit Ethylenoxid, Propylenoxid und/oder Butylenoxid durchgeführt werden. Als Tenside einsetzbar sind hierbei sämtliche alkoxylierten Alkohole, die vorzugsweise mindestens zwei Moleküle eines vorstehend genannten Alkylenoxids addiert enthalten. Auch hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Pro Mol Alkohol verwendet man 2 bis 50, vorzugsweise 3 bis 20 Mol mindestens eines Alkylenoxids. Vorzugsweise setzt man als Alkylenoxid Ethylenoxid ein. Die Alkohole haben vorzugsweise 10 bis 18 Kohlenstoffatome. Je nach Art des Alkoxylierungskatalysators kann man Alkoxylate mit breiter oder enger Alkylenoxid-Homologen-Verteilung erhalten.

Eine weitere Klasse geeigneter nichtionischer Tenside sind Alkylphenolalkoxylate wie Alkylphenolethoxylate mit $C_6$ bis $C_{14}$-Alkylketten und 5 bis 30 Mol Alkylenoxideinheiten.
Eine andere Klasse nichtionischer Tenside sind Alkylpolyglucoside mit 6 bis 22, vorzugsweise 8 bis 18 Kohlenstoffatomen in der Alkylkette. Diese Verbindungen enthalten meist 1 bis 20, vorzugsweise 1 bis 5 Glucosideinheiten.
Eine andere Klasse nichtionischer Tenside sind N-Alkylglucamide der allgemeinen Strukturen

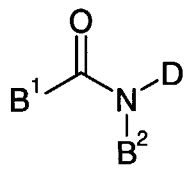

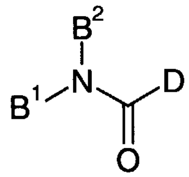

wobei $B^1$ ein $C_6$- bis $C_{22}$-Alkyl, $B^2$ Wasserstoff oder $C_1$- bis $C_4$-Alkyl und D ein Polyhydroxyalkyl-Rest mit 5 bis 12 C-Atomen und mindestens 3 Hydroxygruppen ist. Vorzugsweise steht $B^1$ für $C_{10}$- bis $C_{18}$-Alkyl, $B^2$ für $CH_3$ und D für einen $C_5$- oder $C_6$-Rest. Beispielsweise erhält man derartige Verbindungen durch die Acylierung von reduzierend aminierten Zuckern mit Säurechloriden von $C_{10}$- bis $C_{18}$-Carbonsäuren.
Weitere in Betracht kommende nichtionische Tenside sind die aus der WO-A 95/11225 bekannten endgruppenverschlossenen Fettsäureamidalkoxylate der allgemeinen Formel

$$R^3\text{-CO-NH-}(CH_2)_n\text{-O-}(A^1O)_m\text{-}R^4$$

in der

- $R^3$ einen $C_5$- bis $C_{21}$-Alkyl- oder Alkenylrest bezeichnet,
- $R^4$ eine $C_1$- bis $C_4$-Alkylgruppe bedeutet,
- $A^1$ für $C_2$- bis $C_4$-Alkylen steht,
- n die Zahl 2 oder 3 bezeichnet und
- m einen Wert von 1 - 6 hat.

Beispiele für solche Verbindungen sind die Umsetzungsprodukte von n-Butyltriglykolamin der Formel $H_2N\text{-}(CH_2\text{-}CH_2\text{-}O)_3\text{-}C_4H_9$ mit Dodecansäuremethylester oder die Reaktionsprodukte von Ethyltetraglykolamin der Formel $H_2N\text{-}(CH_2\text{-}CH_2\text{-}O)_4\text{-}C_2H_5$ mit einem handelsüblichen Gemisch von gesättigten $C_8$- bis $C_{18}$-Fettsäuremethylestern.
Weiterhin eignen sich als nichtionische Tenside noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.
Die zusätzlichen nichtionischen Tenside liegen in den erfindungsgemäßen Wasch- und Reinigungsmitteln vorzugsweise in einer Menge von 0.01 bis 40 Gew.-%, insbesondere 0.1 bis 35 Gew.-%, vor allem 0.5 bis 30 Gew.-%, vor.
Man kann einzelne nichtionische Tenside oder eine Kombination unterschiedlicher nichtionischer Tenside einsetzen. Es können nichtionische Tenside aus nur einer Klasse zum Einsatz gelangen, insbesondere nur alkoxylierte $C_8$- bis $C_{22}$-Alkohole, man kann aber auch Tensidmischungen aus verschiedenen Klassen verwenden.
Die erfindungsgemäßen Alkylethersulfate können auch im Gemisch mit weiteren anionischen Tensiden eingesetzt werden. Als weitere anionische Tenside eignen sich $C_8$-bis $C_{24}$-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbernsteinsäuren, Alkenylbernsteinsäuren oder deren Halbester oder Halbamide, Alkylsulfobernsteinsäuren oder deren Amide, Mono- und Diester von Sulfobernsteinsäuren, Acylsarkosinate, sulfatierte Alkylpolyglucoside, Alkylpolyglykolcarboxylate sowie Hydroxyalkylsarkosinate.

Die anionischen Tenside werden dem Wasch- und Reinigungsmittel vorzugsweise in Form von Salzen zugegeben. Geeignete Kationen in diesen Salzen sind Alkalimetallionen wie Natrium, Kalium und Lithium und Ammoniumsalze wie z.B. Hydroxyethylammonium-, Di(hydroxyethyl)ammonium- und Tri(hydroxyethyl)ammoniumsalze.

Die anionischen Tenside liegen in den erfindungsgemäßen Reinigungsmitteln in einer Menge von bis zu 40 Gew.-%, vor allem bis zu 35 Gew.-%, insbesondere bis zu 30 Gew.-% vor. Werden $C_9$- bis $C_{20}$-linear-Alkyl-benzolsulfonate (LAS) mit verwendet, kommen diese üblicherweise in einer Menge bis zu 25 Gew.-%, insbesondere bis zu 20 Gew.-%, zum Einsatz.

Man kann einzelne anionische Tenside oder eine Kombination unterschiedlicher anionischer Tenside einsetzen.

Ferner können die erfindungsgemäß einzusetzenden Alkylethersulfate der allgemeinen Formel I mit kationischen Tensiden, üblicherweise in einer Menge bis zu 25 Gew.-%, vorzugsweise 0.1 bis 15 Gew.-%, beispielsweise $C_8$-bis $C_{16}$-Dialkyldimethylammoniumhalogeniden, Dialkoxydimethylammoniumhalogeniden oder Imidazoliniumsalzen mit langkettigem Alkylrest; und/oder mit amphoteren Tensiden, üblicherweise in einer Menge bis zu 15 Gew.-%, vorzugsweise 0.1 bis 10 Gew.-%, beispielsweise Derivaten von sekundären oder tertiären Aminen wie z.B. $C_6$-$C_{18}$-Alkylbetainen oder $C_6$-$C_{15}$-Alkylsulfobetainen oder Aminoxiden wie Alkyldimethylaminoxiden kombiniert werden.

In der Regel werden die erfindungsgemäß einzusetzenden Alkylethersulfate der allgemeinen Formel I mit Buildern (Sequestrierungsmitteln) wie z.B. Polyphosphaten, Polycarboxilaten, Phosphonaten, Komplexbildnern, z.B. Methylglycindiessigsäure und deren Salze, Nitrilotriessigsäure und deren Salze, Ethylendiamintetraessigsäure und deren Salze sowie gegebenenfalls mit Co-Buildern kombiniert.

Einzelne zur Kombination mit den erfindungsgemäß einzusetzenden Alkylethersulfaten der allgemeinen Formel I gut geeignete Buildersubstanzen seien im Folgenden aufgezählt:

Geeignete anorganische Builder sind vor allem kristalline oder amorphe Alumosilicate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in der US-A-4,604,224.

Als Builder geeignete kristalline Silicate sind beispielsweise Disilicate oder Schichtsilicate, z.B. $\delta$-$Na_2Si_2O_5$ oder ß-$Na_2Si_2O_5$ (SKS 6 bzw. SKS 7). Die Silicate können in Form ihrer Alkalimetall-, Erdalkalimetall- oder Ammoniumsalze eingesetzt werden, vorzugsweise als Na-, Li- und Mg-Silicate. Amorphe Silicate wie beispielsweise Natriummetasilicat, welches eine polymere Struktur aufweist, oder amorphes Disilicat (Britesil® H 20 Hersteller: Akzo) sind ebenfalls verwendbar.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt.

Übliche, als anorganische Builder eingesetzte Phosphate sind Alkali-orthophosphate, und/oder -Polyphosphate wie z.B. Pentanatriumtriphosphat.

Die genannten Builder-Komponenten können einzeln oder in Mischungen untereinander eingesetzt werden.

In einer bevorzugten Ausführungsform enthalten die die erfindungsgemäßen Alkylethersulate der allgemeinen Formel I enthaltenden Wasch- und Reinigungsmittel zusätzlich zu den anorganischen Buildern 0.05 bis 20 Gew.-%, insbesondere 0.1 bis 10 Gew.-% organische Co-Builder in Form von niedermolekularen, oligomeren oder polymeren Carbonsäuren, insbesondere Polycarbonsäuren, oder Phosphonsäuren oder deren Salzen, insbesondere Na- oder K-Salzen.

Als organische Co-Builder geeignete niedermolekulare Carbonsäuren oder Phosphonsäuren sind beispielsweise:

Phosphonsäuren wie z.B. 1-Hydroxyethan-1,1-diphosphonsäure, Amino-tris(methyle-nphosphonsäure), Ethylendiamin-tetra(methylenphosphonsäure), Hexamethylendiamin-tetra(methylenphosphonsäure) und Diethylentriaminpenta(methylenphosphonsäure);

$C_4$-bis $C_{20}$-Di-, -Tri- und -Tetracarbonsäuren wie z.B. Bernsteinsäure, Propantricarbonsäure, Butantetracarbonsäure, Cyclopentantetracarbonsäure und Alkyl- und Alkenylbernsteinsäuren mit $C_2$- bis $C_{16}$-Alkyl- bzw. -Alkenyl-

Resten; $C_4$- bis $C_{20}$-Hydroxycarbonsäuren wie z.B. Äpfelsäure, Weinsäure, Gluconsäure, Glutarsäure, Citronensäure, Lactobionsäure und Saccharosemonodi- und tricarbonsäure; Aminopolycarbonsäuren wie z.B. Nitrilotriessigsäure, ß-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethylendiamindibernsteinsäure und N-(2-Hydroxyethyl)-iminodiessigsäure, Methyl- und Ethylglycindiessigsäure.

Als organische Co-Builder geeignete oligomere oder polymere Carbonsäuren sind beispielsweise:

Oligomaleinsäuren, wie sie beispielsweise in EP-A 451508 und EP-A 396303 beschrieben sind; Co- und Terpolymere ungesättigter $C_4$- bis $C_8$-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% polymerisiert sein können.

Als ungesättigte $C_4$- bis $C_8$-Dicarbonsäuren sind hierbei beispielsweise Maleinsäure, Fumarsäure, Itaconsäure und Citraconsäure geeignet. Bevorzugt wird Maleinsäure.

Die Gruppe (i) umfasst monoethylenisch ungesättigte $C_3$-$C_8$-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfasst monoethylenisch ungesättigte $C_2$- bis $C_{22}$-Olefine, Vinylalkylether mit $C_1$- bis $C_8$-Alkylgruppen, Styrol, Vinylester von $C_1$- bis $C_8$-Carbonsäuren, (Meth)acrylamid und Vinylpyrrolidon. Bevorzugt werden aus der Gruppe (ii) $C_2$- bis $C_6$-Olefine, Vinylalkylether mit $C_1$- bis $C_4$-Alkylgruppen, Vinylacetat und Vinylpropionat eingesetzt.

Falls die Polymere der Gruppe (ii) Vinylester polymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt.

Die Gruppe (iii) umfasst (Meth)acrylester von $C_1$- bis $C_8$-Alkoholen, (Meth)acrylnitril, (Meth)acrylamide von $C_1$- bis $C_8$-Aminen, N-Vinylformamid und N-Vinylimidazol.

Als organische Co-Builder eignen sich auch Homopolymere der monoethylenisch ungesättigten $C_3$-$C_8$-Monocarbonsäuren wie z.B. Acrylsäure, Methacrylsäure, Crotonsäure und Vinylessigsäure, insbesondere der Acrylsäure und Methacrylsäure, Copolymere von Dicarbonsäuren, wie z.B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000; Terpolymere aus Maleinsäure, Acrylsäure und einem Vinylester einer $C_1$-$C_3$-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann; Copolymere von Maleinsäure mit $C_2$-$C_8$-Olefinen im Molverhältnis 40:60 bis 80:20, wobei Copolymere von Maleinsäure mit Ethylen, Propylen, Isobuten oder Diisobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls als organische Co-Builder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere polymerisiert vorliegen. Geeignete modifizierende Monomere sind die oben genannten Monomere der Gruppen (ii) und (iii).

Als Pfropfgrundlage sind abgebaute Polysaccharide wie z.B. sauer oder enzymatisch abgebaute Stärken, Inuline oder Zellulose, Eiweißhydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglycole mit Molmassen mit bis zu $M_w$ = 5000 wie z.B. Polyethylenglycole, Ethylenoxid/Propylenoxid- bzw. Ethylenoxid/Butylenoxid bzw. Ethylenoxid/Propylenoxid/Butylenoxid-Blockcopolymere und alkoxylierte ein- oder mehrwertige $C_1$- bis $C_{22}$-Alkohole.(vgl. US-A-5,756,456)

Als organische Co-Builder geeignete Polyglyoxylsäuren sind beispielsweise beschrieben in EP-B-001004, US-A-5,399,286, DE-A-4106355 und EP-A-656914. Die Endgruppen der Polyglyoxylsäuren können unterschiedliche Strukturen aufweisen.

Als organische Co-Builder geeignete Polyamidocarbonsäuren und modifizierte Polyamidocarbonsäuren sind beispielsweise bekannt aus EP-A-454126, EP-B-511037, WO-A-94/01486 und EP-A-581452.

Als organische Co-Builder verwendet man insbesondere auch Polyasparaginsäuren oder Cokondensate der Asparaginsäure mit weiteren Aminosäuren, $C_4$- bis $C_{25}$-Mono- oder -Dicarbonsäuren und/oder $C_4$- bis $C_{25}$-Mono- oder -Diaminen. Besonders bevorzugt werden in phosphorhaltigen Säuren hergestellte, mit $C_6$- bis $C_{22}$-Mono- oder - Dicarbonsäuren bzw. mit $C_6$- bis $C_{22}$-Mono- oder -Diaminen modifizierte Polyasparaginsäuren eingesetzt.

Als organische Co-Builder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-$\alpha$-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Co-Builder verwendet werden.

In einer weiteren bevorzugten Ausführungsform enthalten die die erfindungsgemäßen Alkylethersulfate enthaltenden Wasch- und Reinigungsmittel zusätzlich, insbesondere zusätzlich zu den anorganischen Buildern, den anionischen Tensiden und/oder den nichtionischen Tensiden, 0.5 bis 20 Gew.-%, insbesondere 1 bis 10 Gew.-%, Glycin-N,N-diessigsäure-Derivate, wie sie in der WO 97/19159 beschrieben sind.

Häufig ist es auch zweckmäßig, den die erfindungsgemäßen Alkylethersulfate enthaltenden Wasch- und Reinigungsmitteln Bleichsysteme, bestehend aus Bleichmittein, wie z.B. Perborat, Percarbonat und gegebenenfalls Bleichaktivatoren, wie z.B. Tetraacetylethylendiamin, + Bleichstabilisatoren zuzusetzen.

In diesen Fällen enthalten die die erfindungsgemäßen Alkylethersulfate enthaltenden Wasch- und Reinigungsmittel zusätzlich 0.5 bis 30 Gew.-%, insbesondere 5 bis 27 Gew.-%, vor allem 10 bis 23 Gew.-% Bleichmittel in Form von Percarbonsäuren, z.B. Diper-oxododecandicarbonsäure, Phthalimidopercapronsäure oder Monoperoxophthalsäure oder -terephthalsäure, Addukte von Wasserstoffperoxid an anorganische Salze, z.B. Natriumperborat-Monohydrat, Natriumperborat-Tetrahydrat, Natriumcarbonat-Perhydrat oder Natriumphosphat-Perhydrat, Addukte von Wasserstoffperoxid an organische Verbindungen, z.B. Harnstoff-Perhydrat, oder von anorganischen Peroxosalzen, z.B. Alkalimetallpersulfaten, oder -peroxodisulfaten, gegebenenfalls in Kombination mit 0 bis 15 Gew.-%, vorzugsweise 0.1 bis 15 Gew.-%, insbesondere 0.5 bis 8 Gew.-%, Bleichaktivatoren.

Als Bleichaktivatoren eignen sich:

- polyacylierte Zucker, z.B. Pentaacetylglucose;
- Acyloxybenzolsulfonsäuren und deren Alkali- und Erdalkalimetallsalze, z.B. Natrium-p-nonanoyloxybenzolsulfonat oder Natrium-p-benzoyloxybenzolsulfonat;
- N,N-diacylierte und N,N,N',N'-tetraacylierte Amine, z.B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonylcarbonamide, z.B. N-Methyl-N-mesylacetamid oder N-Methyl-N-mesylbenzamid;
- N-acylierte cyclische Hydrazide, acylierte Triazole oder Urazole, z.B. Monoacetylmaleinsäurehydrazid;
- O,N,N-trisubstituierte Hydroxylamine, z.B. O-Benzoyl-N,N-succinylhydroxylamin, O-Acetyl-N,N-succinylhydroxylamin oder O,N,N-Triacetylhydroxylamin;
- N,N'-Diacylsulfurylamide, z.B. N,N'-Dimethyl-N,N'-diacetylsulfurylamid oder N,N'-Diethyl-N,N'-dipropionyisulfurylamid;
- acylierte Lactame wie beispielsweise Acetylcaprolactam, Octanoylcaprolactam, Benzoylcaprolactam oder Carbonylbiscaprolactam;
- Anthranilderivate wie z.B. 2-Methylanthranil oder 2-Phenylanthranil;
- Triacylcyanurate, z.B. Triacetylcyanurat oder Tribenzoylcyanurat;
- Oximester und Bisoximester wie z.B. O-Acetylacetonoxim oder Bisisopropyliminocarbonat;
- Carbonsäureanhydride, z.B. Essigsäureanhydrid, Benzoesäureanhydrid, m-Chlorbenzoesäureanhydrid oder Phthalsäureanhydrid;
- Enolester wie z.B. Isopropenylacetat;
- 1,3-Diacyl-4,5-diacyloxy-imidazoline, z.B. 1,3-Diacetyl-4,5-diacetoxyimidazolin;
- Tetraacetylglycoluril und Tetrapropionylglycoluril; diacylierte 2,5-Diketopiperazine, z.B. 1,4-Diacetyl-2,5-diketopiperazin;
- ammoniumsubstituierte Nitrile wie z.B. N-Methylmorpholiniumacetonitrilmethylsulfat;
- Acylierungsprodukte von Propylendiharnstoff und 2,2-Dimethylpropylendiharnstoff, z.B. Tetraacetylpropylendiharnstoff;
- $\alpha$-Acyloxypolyacylmalonamide, z.B. $\alpha$-Acetoxy-N,N'-diacetylmalonamid;
- Diacyl-dioxohexahydro-1,3,5-triazine, z.B. 1,5-Diacetyl-2,4-dioxohexahydro-1,3,5-triazin;
- Benz-(4H)1,3-oxazin-4-one mit Alkylresten, z.B. Methyl, oder aromatischen Resten z.B. Phenyl, in der 2-Position.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise beschrieben sind in US-A 5,360,569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Reinigungsmitteln höchstens in Mengen bis 1.5 Gew.-%, insbesondere bis 0.5 % Gew.-%, im Falle von sehr aktiven Mangankomplexen in Mengen bis zu 0.1 Gew.-%, eingearbeitet.

Neben dem beschriebenen Bleichsystem aus Bleichmitteln, Bleichaktivatoren und gegebenenfalls Bleichkatalysatoren ist für die die erfindungsgemäßen Alkylethersulfate der allgemeinen Formel I enthaltenden Wasch- und Reinigungsmittel auch die Verwendung von Systemen mit enzymatischer Peroxidfreisetzung oder von photoaktivierten Bleichsystemen möglich.

Für eine Reihe von Anwendungsfällen ist es zweckmäßig, dass die die erfindungsgemäßen Alkylethersulfate der allgemeinen Formel I enthaltenden Wasch- und Reinigungsmittel Enzyme enthalten. Vorzugsweise in Wasch- und Reinigungsmitteln eingesetzte Enzyme sind Proteasen, Amylasen, Lipasen und Cellulasen. Von den Enzymen werden vorzugsweise Mengen von 0.1 bis 2.5 Gew.-%, insbesondere vorzugsweise 0.2 bis 1.5 Gew.-%, des konfektionierten Enzyms zugesetzt. Geeignete Proteasen sind z.B. Savinase und Esperase (Hersteller: Novo Nordisk). Eine geeignete Lipase ist z.B. Lipolase (Hersteller: Novo Nordisk). Eine geeignete Cellulase ist z.B. Celluzym (Hersteller: Novo Nordisk). Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann das die erfindungsgemäßen Alkylethersulfate enthaltende Wasch- und Reinigungsmittel noch Enzymstabilisatoren, z.B. Calciumpropionat, Natriumformiat oder Borsäuren oder deren Salze, und/oder Oxidationsverhinderer enthalten.

Die Bestandteile von Wasch- und Reinigungsmitteln sind dem Fachmann prinzipiell bekannt. Die obigen und die weiter unten folgenden Listen geeigneter Bestandteile geben nur einen exemplarischen Ausschnitt der bekannten geeigneten Bestandteile wieder.

Die die erfindungsgemäßen Alkylethersulfate der allgemeinen Formel I enthaltenden Wasch- und Reinigungsmittel können neben den bisher genannten Hauptkomponenten noch folgende weitere übliche Zusätze in den hierfür üblichen Mengen enthalten:

Bekannte Dispergiermittel, wie z.B. Naphthalinsulfonsäurekondensate oder Polycarboxilate, pH-regulierende Verbindungen wie z.B. Alkalien (NaOH, KOH, Pentanatriummetasilikat) oder Säuren (Salzsäure, Phosphorsäure, Amidoschwefelsäure, Citronensäure) Puffersysteme, wie z.B. Acetat oder Phosphatpuffer, Parfüm, Farbstoffe, Biozide, wie z.B. Isothiazolinone oder 2-Bromo-2-nitro-1,3-propandiol, Solubilisatoren/Hydrotrope, wie z.B. Cumolsulfonate, Toluolsulfonate, kurzkettige Fettsäuren, Harnstoff, Alkohole oder Phosphorsäurealkyl/-arylester, Alkyl/Aryl-polyglycolphosphorsäureester, Lösemittel, wie z.B. kurzkettige Alkyloligoglykole wie Butylglykol, Butyldiglykol, Propylenglykolmonomethylether, Alkohole wie Ethanol, i-Propanol, aromatische Lösungsmittel wie Toluol, Xylol, N-Alkylpyrrolidone oder Alkylencarbonate, Verdicker, wie z.B. Polysaccharide, und/oder schwach vernetzte Polycarboxylate (beispielsweise Carbopol® der Firma Goodrich), feinteilige Abrasivkomponenten, wie z.B. Quarz- oder Marmormehl, Kreide, Diatomeenerde, Bimsstein oder auch Polierrot oder Schmirgel, Schaumregulatoren zur Stabilisierung oder Dämpfung des Schaums, Haut- und Korrosionsschutzmittel, desinfizierende Verbindungen oder Systeme, wie z.B. solche die Chlor oder unterchlorige Säure freisetzen wie z.B. Dichlorisocyanurat oder die Jod enthalten.

Die Wasch- und Reinigungsmittel sind gewöhnlich, aber nicht ausschließlich, wässrig und liegen in der Form von Mikroemulsionen, Emulsionen oder Lösungen vor.

Sollten sie in fester, pulverförmiger Form vorliegen, können zusätzlich übliche Stellmittel, die ihnen eine gute Rieselfähigkeit, Dosierbarkeit und Löslichkeit verleihen und/oder die das Zusammenbacken und Stauben verhüten, wie z.B. Natriumsulfat oder Magnesiumsulfat eingesetzt werden.

Bei tablettenförmigen Wasch- und Reinigungsmitteln werden zusätzlich Tablettierhilfsmittel wie z.B. Polyethylenglykole mit Molmassen > 1000 g/mol, Polymerdispersionen, und Tablettensprengmittel wie z.B. Cellulosederivate, vernetztes Polyvinylpyrrolidon, vernetzte Polyacrylate oder Kombinationen aus Säuren und Basen, z.B. Citronensäure und Natriumbicarbonat, um nur einige zu nennen, benötigt.

Die die erfindungsgemäßen Alkylethersulfate der allgemeinen Formel I enthaltenden Wasch- und Reinigungsmittel sind in Ihrer Reinigungswirkung vergleichbaren Wasch- und Reinigungsmitteln überraschenderweise erheblich überlegen.

Darüber hinaus betrifft die vorliegende Erfindung die Verwendung der erfindungsgemäßen Alkylethersulfate der allgemeinen Formel I in chemisch-technischen Anwendungen.

Insbesondere betrifft die vorliegende Erfindung die Verwendung der erfindungsgemäßen Alkylethersulfate der allgemeinen Formel I in Textil-, Papier- und Lederhilfsmitteln, Feuerlöschschäumen, Pestizidformulierungen, in Emulsionspolymerisationen, zur Metallvorbehandlung, als Hilfsmittel für die keramische Industrie, als Kühlschmiermittel oder in Emulgierprozessen.

Die vorliegende Erfindung betrifft weiterhin die Verwendung der erfindungsgemäßen Alkylethersulfate der allgemeinen Formel I in kosmetischen Anwendungen.

Insbesondere betrifft die vorliegende Erfindung die Verwendung der erfindungsgemäßen Alkylethersulfate der allgemeinen Formel I in Duschgelen, Haarshampoos, Badezusätzen, Syndets, Lotions, Ölen/Parfümölen, flüssigen Handwaschseifen und Emulgatoren für Cremes.

Die vorliegende Erfindung betrifft weiterhin Wasch- und Reinigungsmittel oder kosmetische Formulierungen enthaltend Alkylethersulfate der allgemeinen Formel I, wobei der Quotient A, wie oben definiert, größer 1 ist.

## Beispiele

Allgemeine Vorschrift für die Sulfatierung von Alkoholen/Alkoholalkoxylaten

Die Alkoholkomponente, deren Zusammensetzung in Tabelle 1 beschrieben ist, wird in einer Rührapparatur vorgelegt und mit Stickstoff inertisiert. Unter starkem Rühren wird eine äquimolare Menge Chlorsulfonsäure innerhalb von 4 Stunden zugetropft. Die Temperatur wird dabei unter 30 °C gehalten. Durch die viskose Lösung wird über Nacht bei Raumtemperatur unter Stickstoff geströmt um restliches HCl auszutreiben. Anschließend wird der Reaktionsansatz in eine äquimolare Menge 50%ige NaOH getropft, so dass die Temperatur nicht über 45 °C steigt. Der pH-Wert wird eventuell mit 50 %iger NaOH bzw. 50 %iger $H_2SO_4$ auf 8 - 9 eingestellt.

| Beispiel | Alkohol | PO (mol) | EO (mol) | Schaum nach EN 1890, 2g/l | cmc (mmol/l) | A |
|---|---|---|---|---|---|---|
| 1 | 2-Propylheptanol | 2 | 0 | 600 | 1,81 | 13,06 |
| Referenz 1 | 2-Propylheptanol | 0 | 0 | 0 | 23,64 | |
| 2 | 2-Propylheptanol | 2 | 1 | 665 | 1,82 | 11,25 |
| Referenz 2 | 2-Propylheptanol | 0 | 1 | 580 | 20,48 | |
| 3 | 2-Propylheptanol | 2 | 3 | 675 | 1,67 | 4,96 |
| Referenz 3 | 2-Propylheptanol | 0 | 3 | 620 | 8,29 | |
| 4 | i-$C_{13}$-Alkohol | 2 | 0 | 655 | 0,27 | 18,96 |
| Referenz 4 | i-$C_{13}$-Alkohol | 0 | 0 | 750 | 5,12 | |
| 5 | i-$C_{13}$-Alkohol | 2 | 1 | 730 | 0,33 | 3,39 |
| Referenz 5 | i-$C_{13}$-Alkohol | 0 | 1 | 1050 | 1,12 | |
| 6 | i-$C_{13}$-Alkohol | 2 | 3 | 720 | 0,22 | 2,00 |
| Referenz 6 | i-$C_{13}$-Alkohol | 0 | 3 | 930 | 0,44 | |
| PO = Propylenoxid, EO = Ethylenoxid, WAS = waschaktive Substanzen, cmc = critical micell concentration; A = cmc(Ref.-Bsp. x)/cmc(Bsp. x) | | | | | | |

Die Werte für die kritische Micellenkonzentration (cmc) in mmol/l werden über Konzentrationsreihen mit der DeNuoy-Methode der Oberflächenspannungsmessung bestimmt.

## Patentansprüche

1. Alkylethersulfate der allgemeinen Formel I

$$RO\text{-}(CH_2\text{-}CHR^1O)_y\text{-}(CH_2CH_2O)_zSO_3^-\ M^+ \qquad (I),$$

mit der Bedeutung

R von 2-Propylheptanol, i-$C_{13}$-Alkoholen oder Mischungen von 2-Propylheptanol und i-$C_{13}$-Alkoholen abgeleiteter Rest,
$R^1$ Methyl,
$M^+$ Kation, ausgewählt aus der Gruppe bestehend aus Alkalimetallen, $NH_4^+$ und $HNR^2_3{}^+$, wobei $R^2$ ausgewählt ist aus der Gruppe bestehend aus linearen oder verzweigten Alkylresten, $CH_2CH_2OH$ und $CH_2CH(OH)CH_3$,
y mittlerer Wert 2,
z mittlerer Wert von 1 oder 3,

für die der Quotient A der kritischen Micellenkonzentration cmc

$$A = \frac{cmc\ (RO\text{-}(CH_2CH_2O)_zSO_3^-\ M^+)}{cmc\ (RO\text{-}CH_2\text{-}CHR^1O)_y\text{-}(CH_2CH_2O)_zSO_3^-\ M^+)} > 1$$

ist.

2. Verwendung von Alkylethersulfaten gemäß Anspruch 1 als Anionentensidkomponente in Wasch- und Reinigungsmitteln.

3. Verwendung nach Anspruch 2, **dadurch gekennzeichnet, dass** die Wasch- und Reinigungsmittel ausgewählt sind aus der Gruppe bestehend aus Pulverwaschmitteln, Kompaktwaschmitteln, Superkompaktwaschmitteln, Waschmittelextrudaten, Waschmittelgelen, Flüssigwaschmitteln, Flüssigwaschmittelkapseln ("pouches"), Flüssigwaschmittelkonzentraten, Handgeschirrspülmitteln, Geschirrspülmitteln für maschinelle Geschirrspüler, Scheuerreinigern oder -milch, Handwaschpasten oder-gelen, Allzweckreinigern, Glasreinigern, Fensterreinigern, Bodenreinigern, Badreinigern, WC-Reinigern, Küchenreinigern, Schlachthausreinigern, Autoshampoos und Metallreinigern.

4. Verwendung von Alkylethersulfaten gemäß Anspruch 1 als Anionentensidkomponente in chemisch-technischen Anwendungen.

5. Verwendung nach Anspruch 4, **dadurch gekennzeichnet, dass** die chemisch-technischen Anwendungen ausgewählt sind aus der Gruppe bestehend aus Tex til-, Papier- und Lederhilfsmitteln, Feuerlöschschäumen, Pestizidformulierungen, Anwendungen in Emulsionspolymerisationen, zur Metallvorbehandlung, als Hilfsmittel für die keramische Industrie, als Kühlschmiermittel und in Emulgierprozessen.

6. Verwendung von Alkylethersulfaten gemäß Anspruch 1 in kosmetischen Anwendungen.

7. Verwendung nach Anspruch 6, **dadurch gekennzeichnet, dass** die kosmetischen Anwendungen ausgewählt sind aus der Gruppe bestehend aus Duschgelen, Haarshampoos, Badezusätzen, Syndets, Lotions, Ölen/Parfümölen, flüssigen Handwaschseifen und Emulgatoren für Cremes.

8. Wasch- und Reinigungsmittel oder kosmetische Formulierungen, enthaltend Alkylethersulfate gemäß Anspruch 1.

**Claims**

1. An alkyl ether sulfate salt of the general formula I

$$RO\text{-}(CH_2\text{-}CHR^1O)_y\text{-}(CH_2CH_2O)_zSO_3\ M^+ \qquad (I),$$

where

R is a radical derived from 2-propylheptanol, $iC_{13}$ alcohols or mixtures of 2-propylheptanol and $iC_{13}$ alcohols,
$R^1$ is methyl,
$M^+$ is a cation, selected from the group consisting of alkali metals, $NH_4^+$ and $HNR^2{}_3^+$, where $R^2$ is selected from the group consisting of unbranched or branched alkyl radicals, $CH_2CH_2OH$ and $CH_2CH(OH)CH_3$,
y has a mean value of 2,
z has a mean value of 1 or 3,

for which the quotient A of the critical micelle concentration cmc

$$A = \frac{cmc\ (RO\text{-}(CH_2CH_2O)_zSO_3^-\ M^+)}{cmc\ (RO\text{-}CH_2\text{-}CHR^1O)_y\text{-}(CH_2CH_2O)_zSO_3^-\ M^+)} \text{ is } > 1.$$

2. The use of alkyl ether sulfate salts according to claim 1 as anion surfactant component in laundry detergents and cleaning compositions.

3. The use according to claim 2, wherein the laundry detergents and cleaning compositions are selected from the group consisting of powder laundry detergents, compact laundry detergents, super compact laundry detergents, laundry detergent extrudates, laundry detergent gels, liquid laundry detergents, liquid laundry detergent pouches, liquid laundry detergent concentrates, hand dishwashing detergents, dishwashing detergents for mechanical dishwashers, scouring cleaners or scouring milk, handwashing pastes or gels, all-purpose cleaners, glass cleaners, window cleaners, floor cleaners, bath cleaners, WC cleaners, kitchen cleaners, slaughterhouse cleaners, car shampoos and metal cleaners.

4. The use of alkyl ether sulfate salts according to claim 1 as anion surfactant component in chemical engineering applications.

5. The use according to claim 4, wherein the chemical engineering applications are selected from the group consisting of textile aids, papermaking aids and leather aids, fire-extinguishing foams, pesticide formulations, uses in emulsion polymerizations, for metal pretreatment, as aids for the ceramics industry, as cooling lubricants and in emulsifying processes.

6. The use of alkyl ether sulfate salts according to claim 1 in cosmetics applications.

7. The use according to claim 6, wherein the cosmetics applications are selected from the group consisting of shower gels, hair shampoos, bath additives, syndets, lotions, oils/perfume oils, liquid handwashing soaps and emulsifiers for creams.

8. A laundry detergent or cleaning composition or cosmetics formulation comprising alkyl ether sulfate salts according to claim 1.

## Revendications

1. Alkyléthersulfates de formule générale I

$$RO\text{-}(CH_2\text{-}CHR^1O)_y\text{-}(CH_2CH_2O)_zSO_3^-\ M^+ \qquad (I),$$

où

R signifie un radical dérivé du 2-propylheptanol, d'i-$C_{13}$-alcools ou de mélanges de 2-propylheptanol et d'i-$C_{13}$-alcools,
$R^1$ signifie méthyle,
$M^+$ signifie un cation, choisi dans le groupe constitué par les métaux alcalins, $NH_4^+$ et $HNR^2_3{}^+$, où $R^2$ est choisi dans le groupe constitué par les radicaux alkyle linéaires ou ramifiés, $CH_2CH_2OH$ et $CH_2CH(OH)CH_3$,
y signifie une valeur moyenne de 2
z signifie une valeur moyenne de 1 ou 3,

pour lesquels le quotient A de la concentration micellaire critique cmc

$$A = \frac{\text{cmc}\ (RO\text{-}(CH_2CH_2O)_zSO_3^-\ M^+)}{\text{cmc}\ (RO\text{-}CH_2\text{-}CHR^1O)_y\text{-}(CH_2CH_2O)_zSO_3^-\ M^+)} > 1$$

2. Utilisation d'alkyléthersulfates selon la revendication 1 comme composant tensioactif anionique dans des agents de lavage et de nettoyage.

3. Utilisation selon la revendication 2, **caractérisée en ce que** les agents de lavage et de nettoyage sont choisis dans le groupe constitué par les agents de lavage en poudre, les agents de lavage compacts, les agents de lavage

supercompacts, les produits extrudés d'agent de lavage, les gels d'agent de lavage, les agents de lavage liquides, les capsules d'agent de lavage liquide ("pouches"), les produits concentrés d'agent de lavage liquide, les produits de vaisselle à la main, les produits de vaisselle pour lave-vaisselle, les produits ou laits de nettoyage abrasifs, les pâtes ou gels de lavage des mains, les produits de nettoyage universels, les produits de nettoyage pour vitres, pour fenêtres, pour sols, pour salles de bain, pour WC, pour cuisines, pour abattoirs, les shampooings pour voiture et les produits de nettoyage de métaux.

**4.** Utilisation d'alkyléthersulfates selon la revendication 1 comme composant tensioactif anionique dans des applications chimico-techniques.

**5.** Utilisation selon la revendication 4, **caractérisée en ce que** les applications chimico-techniques sont choisies dans le groupe constitué par les adjuvants pour textile, pour papier et pour cuir, les mousses anti-incendie, les formulations pesticides, les applications dans les polymérisations en émulsion, pour le prétraitement de métaux, comme adjuvants pour l'industrie céramique, comme lubrifiants à froid et dans les procédés d'émulsion.

**6.** Utilisation d'alkyléthersulfates selon la revendication 1 dans des applications cosmétiques.

**7.** Utilisation selon la revendication 6, **caractérisée en ce que** les applications cosmétiques sont choisies dans le groupe constitué par les gels douche, les shampooings pour cheveux, les sels de bain, les Syndets, les lotions, les huiles/huiles parfumées, les savons liquides pour les mains et les émulsifiants pour crèmes.

**8.** Agent de lavage et de nettoyage ou formulations cosmétiques contenant des alkyléthersulfates selon la revendication 1.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1354872 A
- WO 0058428 A
- WO 9965972 A
- WO 9515408 A
- JP 06017089 B
- JP 06017088 B
- US 3843706 A
- US 3462525 A
- US 3420875 A
- US 3524864 A
- WO 9511225 A
- US 4604224 A
- EP 451508 A
- EP 396303 A
- US 3887806 A
- DE 4313909 A
- US 5227446 A
- DE 4415623 A
- US 5756456 A
- EP 001004 B
- US 5399286 A
- DE 4106355 A
- EP 656914 A
- EP 454126 A
- EP 511037 B
- WO 9401486 A
- EP 581452 A
- WO 9719159 A
- US 5360569 A
- EP 453003 A
- WO 9421777 A


### In der Beschreibung aufgeführte Nicht-Patentliteratur


- **WEIL, JAMES K. et al.** Oxypropylation of fatty alcohols and the sulfation products. *Journal of the American Oil Chemists' Society,* 1966, vol. 43, 157-160
- **CHINODA, KOZO et al.** Ionic surfactants soluble in hard water and in hydrocarbons: behaviour of organized surfactant solutions as a function of the hydrophiliclipophilic balance. *Journal of Physical Chemistry,* 1986, vol. 90 (7), 1228-1230
- **WEIL, JAMES K. et al.** Synthesis and surface active properties of long-chain ether alcohol sulfates. *Chim. Phys. Appl. Prat. Ag. Surface, C. R. Congr. Int. Deterg., 5th, Meeting Date 1968,* 1968, vol. 1, 45-50
- Ullmann's Encyclopedia of Industrial Chemistry. 1994, vol. A25, 779-783
- Ullmann's Encyclopedia of Industrial Chemistry. 1986, vol. A7, 137 ff